Europäisches Patentamt

European Patent Office

Office européen des brevets

(11) Numéro de publication: **0 159 260**
**B1**

# FASCICULE DE BREVET EUROPEEN

(45) Date de publication du fascicule du brevet:
**11.05.88**

(21) Numéro de dépôt: **85400636.8**

(22) Date de dépôt: **01.04.85**

(51) Int. Cl.⁴: **A 61 M 1/00**

(54) **Dispositif d'accès atraumatique au circuit sanguin.**

(30) Priorité: **02.04.84 FR 8405141**

(43) Date de publication de la demande:
**23.10.85 Bulletin 85/43**

(45) Mention de la délivrance du brevet:
**11.05.88 Bulletin 88/19**

(84) Etats contractants désignés:
**AT BE CH DE FR GB IT LI LU NL SE**

(56) Documents cités:
**WO - A - 81/03424**
**FR - A - 2 387 044**
**GB - A - 2 073 027**

(73) Titulaire: **BIOMASYS Société dite:, Château de Beauregard Nan-Sous-Thil, F-21390 Precy-sous-Thil (FR)**

(72) Inventeur: **Lapeyre, Didier, Chaignes, F-27120 Pacy Sur Eure (FR)**
Inventeur: **Slonina, Jean-Pierre, 23 bis, route de la Cascade, F-78110 Le Vesinet (FR)**

(74) Mandataire: **Orès, Bernard et al, Cabinet ORES 6, Avenue de Messine, F-75008 Paris (FR)**

ACTORUM AG

## Description

La présente invention est relative à un dispositif d'accès atraumatique au circuit sanguin, du type comportant essentiellement un dispositif implantable de prélèvement percutané du sang et un mécanisme de commande de l'écoulement sanguin, permettant d'effectuer, aussi souvent qu'il est nécessaire, une circulation extra-corporelle destinée à compenser un mauvais fonctionnement des reins notamment.

On connaît déjà un dispositif d'accès au circuit sanguin comportant un dispositif percutané en forme de T qui est réalisé en une seule pièce en un matériau biologiquement compatible, tel que le titane:

- dont les segments latéraux du T présentent chacun une crête annulaire de retenue et peuvent être insérés directement dans un vaisseau sanguin, préalablement fendu longitudinalement et ensuite suturé ou, en alternative, peuvent être insérés au milieu d'une canalisation sanguine artificielle, notamment réalisée en polytétrafluoréthylène expansé, en établissant un shunt entre une artère et une veine d'assez gros diamètres, et

- dont le segment central du T comporte un élément d'obturation assurant l'étanchéité par rapport aux segments latéraux et constitué par une cloison circulaire transversale, en élastomère de silicone notamment, qui comporte deux fentes ménagées sur un même diamètre, à partir du bord de la cloison, et séparées d'une partie pleine centrale, dans lesquelles fentes peut être introduit un dispositif à aiguilles ou à canules, relié au système d'épuration du sang, dont le centrage par rapport auxdites fentes est obtenu par deux dépressions hémisphériques centrées autour de ces fentes.

Ces dernières sont maintenues serrées par l'intermédiaire d'une bague toroïdale de serrage, en élastomère, insérée dans une rainure circulaire ménagée dans l'épaisseur de ladite cloison, ce qui assure l'étanchéité fonctionnelle nécessaire.

Cette cloison est maintenue en position à l'aide d'une plaquette transversale de compression réalisée en une seule pièce avec une bague tubulaire de compression, cette dernière étant insérée à force dans la cavité du segment central du T et étant maintenue en position à l'aide de deux projections qui font saillie vers l'extérieur à partir de son bord supérieur et qui s'encliquettent dans une rainure circulaire ménagée à proximité du bord correspondant du segment central du T.

La plaquette transversale de pression présente deux orifices correspondant auxdites deux fentes et sépare, dans la bague de presison, une chambre supérieure d'une chambre inférieure: dans cette dernière est emprisonné à force la partie supérieure de ladite cloison d'étanchéité, et se projète un ergot excentré d'alignement entre lesdites fentes et lesdits orifices, qui fait saillie de la surface inférieure de ladite plaquette et qui est logé dans une cavité correspondante de la cloison élastomère.

Ledit dispositif percutané peut présenter un revêtement de carbone pyrolytique, pour augmenter la biocompatibilité; en outre, il peut présenter, sauf sur la partie supérieure du segment central du T, un revêtement en matériau poreux, tel que le téréphtalate de polyéthylène ou le titane poreux, constituant une structure de support colonisable par croissance de tissus.

Ladite bague tubulaire d'étanchéité est réalisée en un métal biocompatible ou, en alternative, par moulage par injection d'une matière plastique telle que du polycarbonate ou du polysulfone, éventuellement renforcée par une charge de verre, de carbone ou d'un minéral.

L'insertion et l'extraction de la cloison d'étanchéité, assemblée avec la bague tubulaire de pression, se fait à l'aide d'outils spécialement conçus à cet effet.

On connaît encore un dispositif d'accès au circuit sanguin qui peut être également connecté à un vaisseau sanguin directement, ou par l'intermédiaire d'un shunt artério-veineux, et qui comporte:

- un dispositif percutané constitué par un conduit conique d'écoulement du sang, réalisé en une matière biocompatible, non poreuse et non biodégradable, notamment en carbone pyrolytique déposé sur un substrat de graphite ou en carbone vitreux, dont la surface externe présente un aspect lisse, lequel conduit comprend à l'extrémité distale une première collerette perforée de fixation et, à la partie intermédiaire, une deuxième collerette perforée l'ancrage biologique par invasion des perforations par croissance de tissus, et

- un élément d'obturation, plein et également conique, dudit conduit d'écoulement, se terminant par une partie proximale non conique améliorant l'étanchéité.

Cet autre dispositif percutané coopère avec:

- un mécanisme atraumatique de commande de l'écoulement sanguin, par l'intermédiaire d'une tige, actionnée de l'extérieur, qui se visse dans ledit élément d'obturation après avoir traversé un orifice ménagé dans une chambre d'obturation du conduit d'écoulement, laquelle chambre est en communication avec un dispositif de traitement du sang par l'intermédiaire d'un tube unique, à travers lequel le sang est alternativement retiré pour être traité et puis renvoyé au corps, ou par l'intermédiaire de deux tubes dont le premier est destiné à retirer le sang et le deuxième à le renvoyer simultanément au corps; ledit mécanisme de commande coopère également avec une bague de fixation de ladite chambre d'obturation contre un rebord dudit conduit d'écoulement, et

- un couvercle, lui aussi coopérant avec une bague de fixation contre ledit rebord du conduit d'écoulement.

Le conduit d'écoulement comporte un revêtement au moins partiel, disposé entre lesdites première et deuxième collerettes de fixation et d'ancrage biologique notamment, réalisé en une matière de greffage vasculaire, en particulier en téréphtalate de polyéthylène, ainsi qu'un revêtement partiel microporeux imperméable au sang, et donc minimisant la perte de sang, qui est appliqué en particulier en correspondance de ladite

2

première collerette de fixation et est réalisé en polytétrafluoréthylène.

Ledit élément d'obturation conique peut présenter également deux canaux séparés par une cloison centrale longitudinale, réliée à la base de cet élément, laquelle base obture le conduit d'écoulement en condition de repos.

Lesdits canaux communiquent chacun, à la partie inférieure, avec un orifice transversal ménagé à proximité de la base d'obturation et, à la partie supérieure, avec une chambre tubulaire d'écoulement reliée à un dispositif de traitement du sang.

Dans ce cas, l'application d'un outil de commande approprié permet d'abaisser l'élément d'obturation, relié auxdites chambres d'écoulement, et de faire plonger la base d'obturation avec lesdits orifices dans le courant sanguin, établissant ainsi la circulation extracorporelle désirée; l'actionnement dudit outil de commande en sens inverse fait remonter l'élément d'obturation avec lesdites chambres tubulaires d'écoulement, ce qui provoque l'obturation desdits orifices et l'arrêt de la circulation extra-corporelle.

En condition de repos la base obture le conduit d'écoulement et l'élément d'obturation présente un certain jeu fonctionnel par rapport aux parois du conduit d'écoulement, lequel jeu est juste suffisant pour permettre son abaissement en position d'ouverture et donc la mise à découvert desdits orifices dans le courant sanguin.

L'art antérieur présente des inconvénients, notamment:

– en ce qui concerne le dispositif percutané:
· la fiabilité et le nettoyage sont aléatoires;
· ledit revêtement poreux colonisable par croissance de tissus présente, à cause des matériaux utilisés jusqu'ici et par rapport aux tissus organiques, une affinité biologique qui est loin d'être totale, ce qui empêche également dans ce cas un ancrage vraiment intime, donc
· la résistance aux efforts accidentels de déchirement exercés à la jonction de la peau est faible, ce qui crée une «marsupialisation» de la peau avec risques d'infection;
– en ce qui concerne le mécanisme de commande de l'écoulement sanguin, celui-ci, comporte de nombreuses pièces souvent difficiles à nettoyer, d'où risque plus grand d'obturation par caillot ou asepsie insuffisante;
– en ce qui concerne le dispositif d'accès au circuit sanguin dans son ensemble, il comporte l'emploi de nombreux outils accessoires qui en compliquent l'usage.

La présente invention a, en conséquence, pour but de pourvoir à un dispositif d'accès atraumatique au circuit sanguin, du type comportant essentiellement un dispositif percutané implantable et un mécanisme de commande de l'écoulement sanguin, qui répond mieux aux nécessités de la pratique que les dispositifs visant au même but antérieurement connus, notamment en ce que:

l'ancrage biologique par croissance de tissus du dispositif percutané est plus intime, ce qui assure:

· une résistance plus élevée aux efforts accidentels de déchirement exercés à la jonction de la peau, et
· une barrière plus efficace contre la pénétration des bactéries dans le siège de l'implantation, éliminant ainsi les risques d'infection,
– l'étanchéité et le nettoyage dudit dispositif percutané sont plus fiables,
– l'actionnement du mécanisme de commande ne nécessite pas d'outils, ce qui le rend particulièrement simple et fiable,
– les pièces dudit mécanisme de commande sont réduites à un minimum et leur conformation est telle que leur nettoyage est très facile et fiable, ce qui élimine le risque d'obturation du courant sanguin par caillot ou asepsie insuffisante.

La présente invention a pour objet un dispositif d'accès atraumatique au circuit sanguin, du type comportant en combinaison:

– un dispositif implantable pourvu d'un segment tubulaire d'accès à un vaisseau sanguin, le dit segment dépassant légèrement de la surface cutanée,
– un élément d'obturation en matière élastique de ce segment,
– un mécanisme de commande de l'écoulement sanguin à travers cet élément d'obturation, ayant une partie proximale et une partie distale, ou, lorsque ce mécanisme est retiré,
– un couvercle de fermeture dudit segment tubulaire d'accès au sang,
lequel dispositif est caractérisé en ce que ledit élément d'obturation élastique est constitué par une pièce tubulaire réalisée en une matière élastique hémocompatible insérée à l'intérieur dudit segment d'accès au sang, lequel élément d'obturation est fermé à sa base et est pourvu d'au moins un orifice latéral de communication avec ledit vaisseau sanguin et présente des moyens de retenue, les orifices latéraux de l'élément d'obturation étant obturés par la paroi de ce segment en l'absence dudit mécanisme de commande et ledit élément d'obturation tubulaire étant allongé élastiquement, lorsque le mécanisme de commande est présent, par la poussée axiale de ladite partie distale de ce dernier sur la base de l'élément d'obturation, de manière à immerger dans le courant sanguin cette base et lesdits orifices, tandis que l'élément d'obturation reprend ses dimensions initiales sous l'action de la contrainte due à son allongement élastique lorsque ledit mécanisme de commande de l'écoulement sanguin est retiré.

Selon un mode de réalisation préféré du dispositif d'accès au circuit sanguin conforme à l'invention, lesdits moyens de retenue de l'élément d'obturation tubulaire sont constitués de préférence par une collerette annulaire ménagée autour de l'ouverture d'accès à cet élément d'obturation, laquelle collerette annulaire est pourvue d'une pluralité d'ergots transversaux de blocage en rotation ménagés dans l'épaisseur de la collerette, et le segment tubulaire du dispositif implantable présente une rainure circulaire dans laquelle est logée ladite collerette de retenue de l'élément d'obturation, lesdits ergots transversaux étant logés à leur tour dans des orifices percés dans la zone de la paroi dudit segment tubulaire qui correspond à ladite rainure circulaire, laquelle rainure circulaire est ménagée dans la paroi interne du segment tubulaire à une distance de l'orifice d'accès à ce segment tubulaire telle que, en l'absence d'application dudit mécanisme de commande ou dudit couvercle, la base dudit élément d'obturation est légèrement rentrante par rapport à la base dudit segment tubulaire.

Selon un mode de réalisation avantageux du dispositif d'accès au circuit sanguin conforme à l'invention, lesdits orifices latéraux du communication avec ledit vaisseau sanguin sont ménagés sensiblement au niveau de l'intersection de la base de fermeture de l'élément d'obturation avec la paroi latérale de ce dernier.

Selon une disposition avantageuse de ce mode de réalisation, lesdits orifices latéraux sont oblongs et ménagés avec l'axe d'écoulement sanguin de ces orifices incliné vers ledit vaisseau sanguin d'un angle aigu, qui de préférence est de 60°, par rapport à l'axe de l'élément d'obturation tubulaire et passant sensiblement par le point d'intersection de ladite base avec ladite paroi latérale.

Selon un autre mode de réalisation avantageux du dispositif d'accès au circuit sanguin conforme à l'invention, la base de fermeture de l'élément d'obturation tubulaire présente extérieurement une configuration arquée destinée à réduire la turbulence due à l'immersion de cette base dans le courant sanguin, laquelle configuration arquée est délimitée transversalement, à savoir perpendiculairement au sens de l'écoulement sanguin, par deux projections latérales sensiblement triangulaires qui sont alignées avec la paroi latérale de l'élément d'obturation.

Selon un autre mode de réalisation avantageux du dispositif d'accès au circuit sanguin conforme à l'invention, lorsque celui-ci est du type comportant un mécanisme de commande de l'écoulement sanguin qui comprend deux chambres tubulaires d'écoulement communiquant avec deux embouts reliés à un dispositif d'épuration sanguine qui sont pourvues de deux encoches diamétralement opposées, notamment en forme de V à grande ouverture, et qui sont solidarisées entre elles par une cloison longitudinale centrale et une enveloppe extérieure commune à ces chambres d'écoulement, l'enveloppe desdites deux chambres d'écoulement comporte une collerette annulaire, ménagée sensiblement à sa partie médiane, par rapport à laquelle ces deux chambres d'écoulement se prolongent bilatéralement dans lesdites parties distale et proximale, laquelle collerette est destinée à coopérer avec des moyens de blocage des chambres tubulaires et d'entraînement par roto-translation de la partie distale de ces dernières à l'intérieur dudit élément d'obturation tubulaire élastique.

Selon une disposition avantageuse de ce mode de réalisation, lesdits moyens de blocage et d'entraînement des chambres tubulaires d'écoulement sont constitués par un écrou moleté, dont le corps cylindrique comporte un premier siège central de logement de ladite collerette faisant saillie de l'enveloppe des chambres d'écoulement, un deuxième siège central de centrage superposé au premier et de plus grand diamètre, et une partie en saillie centrale, disposée du côté opposé auxdits premier et deuxième sièges, laquelle est en communication avec ces derniers et filetée sur sa surface extérieure destinée à être vissée dans une partie taraudée dudit segment tubulaire du dispositif implantable se trouvant au-dessus de ladite rainure circulaire, ainsi que par une bague de fixation, coaxiale et superposée par rapport audit écrou, qui se compose de deux demi-bagues comportant chacune, sur une face transversale, une rainure semi-circulaire délimitant centralement une partie destinée à être logée et centrée dans ledit deuxième siège de centrage de cette bague composite et à emprisonner, avec ledit écrou, ladite collerette des chambres d'écoulement dans ledit premier siège, tandis que latéralement ladite rainure semi-circulaire délimite une partie destinée à envelopper partiellement ledit écrou, la solidarisation entre les deux demi-bagues de fixation et l'écrou étant obtenue à l'aide de vis notamment.

Selon un autre mode de réalisation avantageux du dispositif d'accès au circuit sanguin conforme à l'invention, ledit couvercle comporte une plaquette d'obturation destinée à couvrir le segment tubulaire du dispositif implantable, ainsi que son revêtement, et une première partie cylindrique centrale qui fait saillie à partir de ladite plaquette et qui est filetée et destinée à être vissée dans ladite partie taraudée du segment tubulaire.

Selon un mode de réalisation préféré du dispositif d'accès au circuit sanguin conforme à l'invention, le couvercle comporte une deuxième partie cylindrique centrale en saillie, de plus petit diamètre par rapport à ladite première partie en saillie filetée, qui se projette, à partir de cette dernière, à l'intérieur dudit élément d'obturation tubulaire et qui est entourée par un manchon de pression se prolongeant jusqu'à la base de cet élément d'obturation tubulaire et étant inséré à force dans ce dernier, lequel manchon est donc immobilisé en rotation et comprime la paroi latérale dudit élément d'obturation contre la paroi dudit segment tubulaire du dispositif implantable, tandis qu'il permet la libre rotation de ladite deuxième partie en saillie à l'intérieur de ce même manchon.

Selon un autre mode de réalisation préféré du dispositif d'accès au circuit sanguin conforme à l'invention, le couvercle présente une troisième partie cylindrique centrale qui fait saillie à partir de ladite deuxième partie en saillie et qui présente un plus petit diamètre par rapport à cette dernière, laquelle troisième partie en saillie est entourée par une rondelle de retenue, de diamètre supérieur à celui de la deuxième partie en saillie, qui est maintenue contre cette dernière par un évasement ou élargissement de l'extrémité distale de ladite troisième partie en saillie, réalisée par sertissage ou par formage à chaud de cette extrémité

7 0159260 8

notamment, laquelle rondelle est logée dans une rainure cylindrique ménagée à la partie distale dudit manchon de pression, dans la paroi interne de ce dernier, et définissant dans cette même paroi un épaulement contre lequel est appliquée ladite rondelle qui tient en position ledit manchon de pression.

Selon un autre mode de réalisation avantageux du dispositif d'accès au circuit sanguin conforme à l'invention, la plaquette d'obturation dudit couvercle comporte un joint d'étanchéité annulaire logé à force dans une rainure circulaire ménagée dans la face d'obturation de la plaquette.

Selon un autre mode de réalisation préféré du dispositif d'accès au circuit sanguin conforme à l'invention, lorsqu'il est du type comportant en outre un revêtement en un matériau poreux colonisable par croissance de tissus et entourant au moins partiellement ledit dispositif implantable, ce revêtement poreux est constitué par un matériau composite carbone-carbone du type comportant une armature constituée par un substrat poreux en fibres de carbone noyée dans une matrice carbonée de densification de ce substrat fibreux et de liaison entre les fibres de carbone, dans lequel matériau composite carbone-carbone le substrat poreux en fibres de carbone est densifié seulement en partie par ladite matrice carbonée de façon à obtenir un matériau composite carbone-carbone poreux.

Selon une disposition avantageuse de ce mode de réalisation, la porosité dudit matériau composite est de préférence comprise entre 40 et 50% de la porosité initiale dudit substrat de fibres de carbone.

Selon un autre mode de réalisation avantageux du dispositif d'accès au circuit sanguin, ledit revêtement poreux entoure complètement au moins ledit segment tubulaire du dispositif implantable sur toute sa longueur, y compris la partie de ce dernier qui dépasse légèrement de l'épiderme, et comporte une partie proximale sensiblement cylindrique, dont la hauteur est légèrement supérieure à l'épaisseur totale du derme et de l'épiderme et qui est suivie d'une partie intermédiaire en forme de collerette circulaire assurant l'ancrage biologique principal et disposée juste sous le derme, et d'une partie distale comportant une pluralité de nervures circulaires assurant un ancrage biologique supplémentaire.

Selon une disposition préférée de ce mode de réalisation, la partie proximale sensiblement cylindrique dudit revêtement poreux en composite carbone-carbone comporte une pellicule en carbone pyrolytique massif, étanche et non poreux, de protection contre la pénétration d'agents nuisibles tels que poussière, liquides ou autres, laquelle pellicule est appliquée sur la portion exposée du revêtement parallèle à l'épiderme ainsi que sur une petite portion de sa surface latérale extérieure perpendiculaire à l'épiderme.

Outre les dispositions qui précédent, l'invention comprend encore d'autres dispositions, qui ressortiront de la description qui va suivre.

L'invention sera mieux comprise à l'aide du complément de description qui va suivre, qui se réfère aux dessins annexés dans lesquels:

La fig. 1 représente une vue en coupe du dispositif d'accès atraumatique au circuit sanguin, comportant un dispositif percutané et un mécanisme de commande de l'écoulement sanguin conformes à l'invention, qui coopèrent pendant que l'opération de dialyse rénale est en cours;

les fig. 2 et 3 sont des vues en coupe, suivant II et III respectivement, du dispositif représenté à la fig. 1 montrant les détails de l'extrémité distale du mécanisme de commande de l'écoulement sanguin coopérant avec l'élément d'obturation conformes à la présente invention;

la fig. 4 représente une vue en coupe du dispositif percutané regardé suivant la flèche IV, coopérant avec le couvercle selon l'invention;

la fig. 5 correspond à la fig. 4 dans laquelle le dispositif percutané est représenté avec l'élément d'obturation selon l'invention en position de repos, à savoir en l'absence dudit mécanisme de commande de l'écoulement sanguin ainsi que dudit couvercle conformes à l'invention;

les fig. 6 à 8 se réfèrent à un mode de réalisation préféré de l'élément d'obturation élastique tubulaire conforme à l'invention, et en particulier:

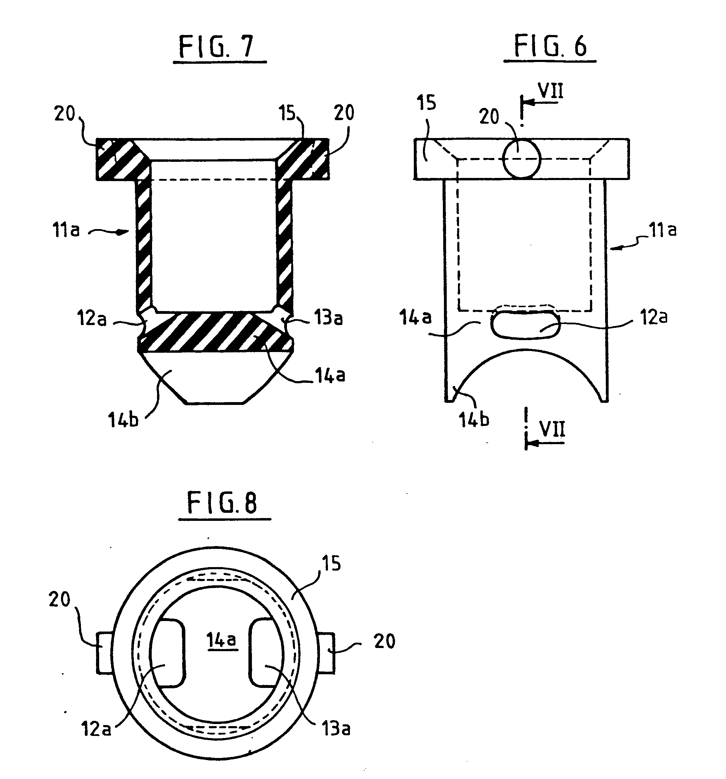

La fig. 6 est une vue en élévation suivant le sens de l'écoulement sanguin,

la fig. 7 est une vue en coupe suivant le plan VII de fig. 6, et

la fig. 8 est une vue de dessus.

Il doit être bien entendu, toutefois, que ces dessins et les parties descriptives correspondantes, sont donnés uniquement à titre d'illustration de l'objet de l'invention, dont ils ne constituent en aucune manière une limitation.

Le dispositif d'accès atraumatique au circuit sanguin selon l'invention est du type comportant un dispositif percutané 1 implantable qui, de préférence, est en forme de T et qui coopère, notamment en cours de dialyse rénale, avec un mécanisme de commande d'écoulement sanguin 2 et, en conditions normales, avec un couvercle 3. Ce dispositif percutané est réalisé en une seule pièce et de préférence en titane. Toutefois, l'emploi de l'acier inoxydable est également envisageable.

En ce qui concerne le revêtement poreux 4 du dispositif percutané 1, on fait usage des techniques de production des matériaux composites cabone-carbone, qui sont bien connues des techniciens en la matière.

Un composite carbone-carbone est constitué par deux éléments:

— un substrat poreux en fibres de carbone servant de renfort, et

— une matrice carbonée de densification du substrat fibreux assurant la liaison entre les fibres de carbone.

La densification de ce substrat poreux est obtenue à l'aide de deux techniques principales notamment:

— par dépôt chimique en phase vapeur de carbone pyrolytique autour desdites fibres de carbone, obtenu par décomposition thermique d'un

5

hydrocarbure gazeux dans des conditions définies de température et de pression (1000 °C environ et 10 torrs environ), les cristallites de la matrice de carbone pyrolytique, ainsi obtenue, étant orientés sensiblement parallèlement à l'axe des fibres de carbone, ou

– par imprégnation dudit substrat fibreux par un liquide organique qui est ensuite carbonisé pour ne laisser subsister qu'un squelette de coke.

Dans le cadre de la présente invention, on est en réalité intéressé à la première des deux techniques qui viennent d'être exposées, étant donné que celle-ci permet de contrôler la densification du substrat fibreux et donc la porosité finale de ce dernier, pour l'adapter à la colonisation des fibroblastes générés par le dessous du derme 5: de préférence la porosité dudit revêtement composite 4 est comprise entre 40 et 50% de la porosité initiale dudit substrat de fibres de carbone, ces dernières étant sensiblement perpendiculaires au derme 5 et à l'épiderme 6.

La densité finale du matériau composite carbone-carbone poreux est de préférence comprise entre 0,8 et 1,2 g/cm³.

Les pores du carbone poreux présentent un diamètre compris entre 12 et 15 μm, ce qui assure, par rapport aux autres revêtements poreux proposés dans l'Art antérieur, tels que le titane poreux ou le téréphtalate de polyéthylène, une colonisation plus intime de la part des fibroblastes donnant lieu à la formation de collagène, avec un ancrage biologique plus efficace.

Ce revêtement composite 4 entoure complètement le segment central 7 du dispositif percutané 1 sur toute sa longueur, y compris la partie de ce dernier qui dépasse légèrement de la peau.

Ledit revêtement composite 4 comporte:

– une partie proximale sensiblement cylindrique 8, dont la hauteur est légèrement supérieure à l'épaisseur totale du derme 5 et de l'épiderme 6;

– une partie intermédiaire en forme de collerette circulaire 9 assurant l'ancrage biologique principal dudit revêtement 4 et disposée juste sous le derme 5, et

– une partie distale comportant une pluralité de nervures circulaires 10 assurant un ancrage biologique supplémentaire.

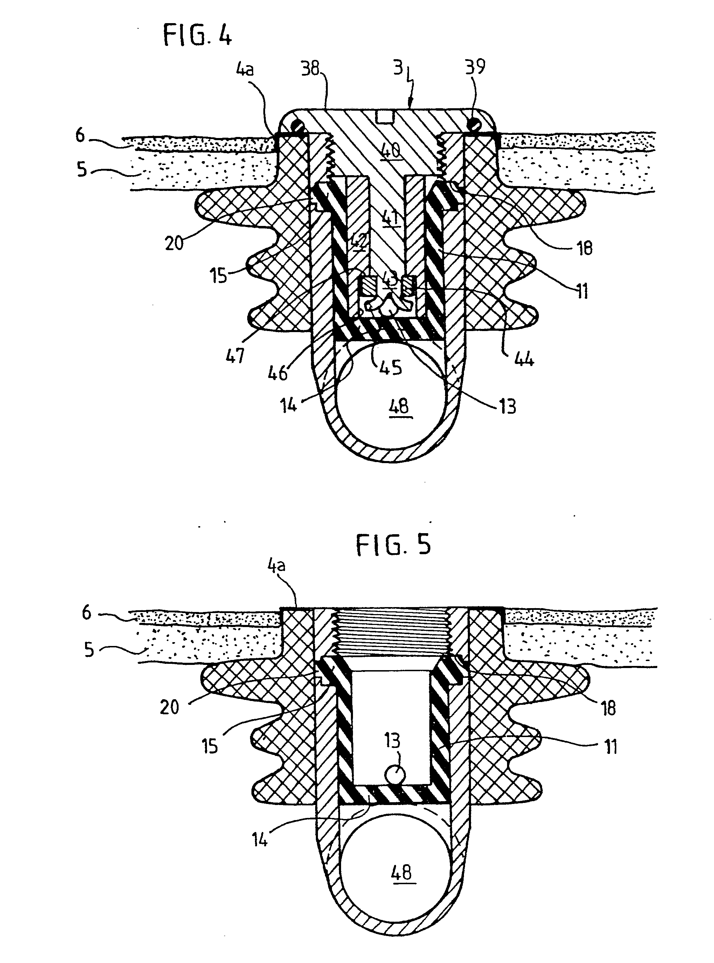

Ladite partie proximale 8 du revêtement composite carbone-carbone poreux peut être avantageusement revêtue, au niveau de la portion exposée parallèle à l'épiderme 6 et de la portion latérale externe perpendiculaire à l'épiderme 6, par une pellicule mince 4a (cf. la fig. 4), dont l'épaisseur est de préférence de l'ordre de quelques centièmes de millimètre, qui est réalisée en carbone pyrolytique massif, étanche et non poreux, pour protéger ledit revêtement 4 de toute imprégnation nuisible, en particulier de la part de poussière, de liquides ou autres.

Ledit dispositif percutané 1 comporte également un élément d'obturation 11 qui, selon l'invention, est constitué par une pièce tubulaire en matière élastique hémo-compatible, tel que le polyuréthane, obtenue par moulage.

Deux orifices 12 et 13 diamétralement opposés, destinés à l'aspiration et la réinjection du sang respectivement, sont ménagés en correspondance de l'extrémité distale dudit élément d'obturation 11, sensiblement au niveau de l'intersection de la base 14 de fermeture de cet élément d'obturation 11 avec la paroi latérale de ce dernier, tandis qu'une collerette annulaire 15 de retenue est présente en correspondance de son extrémité proximale. En l'absence dudit mécanisme de commande de l'écoulement sanguin 2, les orifices 12 et 13 ne débouchent pas dans le courant sanguin délimité par les segments latéraux 16 et 17 du dispositif percutané 1.

Les deux crêtes annulaires 16a et 17a favorisent la solidarisation entre le dispositif percutané et le shunt artério-veineux sur lequel il est disposé: en particulier ces crêtes empêchent le glissement de shunt.

L'étanchéité est alors assurée (cf. la fig. 4) par la base 14 de l'élément d'obturation tubulaire 11 qui est par ailleurs inséré à force dans ledit segment central 7.

Ladite collerette 15 est logée dans une rainure circulaire 18 qui est ménagée dans la paroi interne du segment central 7 du dispositif percutané 1 à une distance de l'orifice d'accès à ce segment central 7 telle que, en l'absence d'application dudit mécanisme de commande 2 ou dudit couvercle 3, à savoir en condition de repos, la base 14 dudit élément d'obturation 11 est nettement au-dessus de la base 19 du segment central 7 (par exemple, de 0,6 à 1 mm), pour éviter qu'avec le vieillissement, à savoir la variation dans le temps des propriétés élastiques du matériau dont se compose l'élément d'obturation 11, ce dernier dépasse ladite base 19, ce qui serait source de formation indésirée de caillots.

Des ergots transversaux 20, venus de moulage, bloquent en rotation la collerette 15 et viennent se loger dans des orifices percés dans la zone de ladite paroi du segment central 7 occupée par ladite raninure circulaire 18.

De préférence, on utilise deux ergots diamétralement opposés ou quatre ergots à 90° l'un par rapport à l'autre; ces ergots 20 permettent également d'orienter parfaitement lesdits orifices d'aspiration et de réinjection 12 et 13 par rapport au courant sanguin.

L'élément d'obturation tubulaire élastique selon l'invention peut prendre de préférence la configuration 11a illustrée aux fig. 6 à 8.

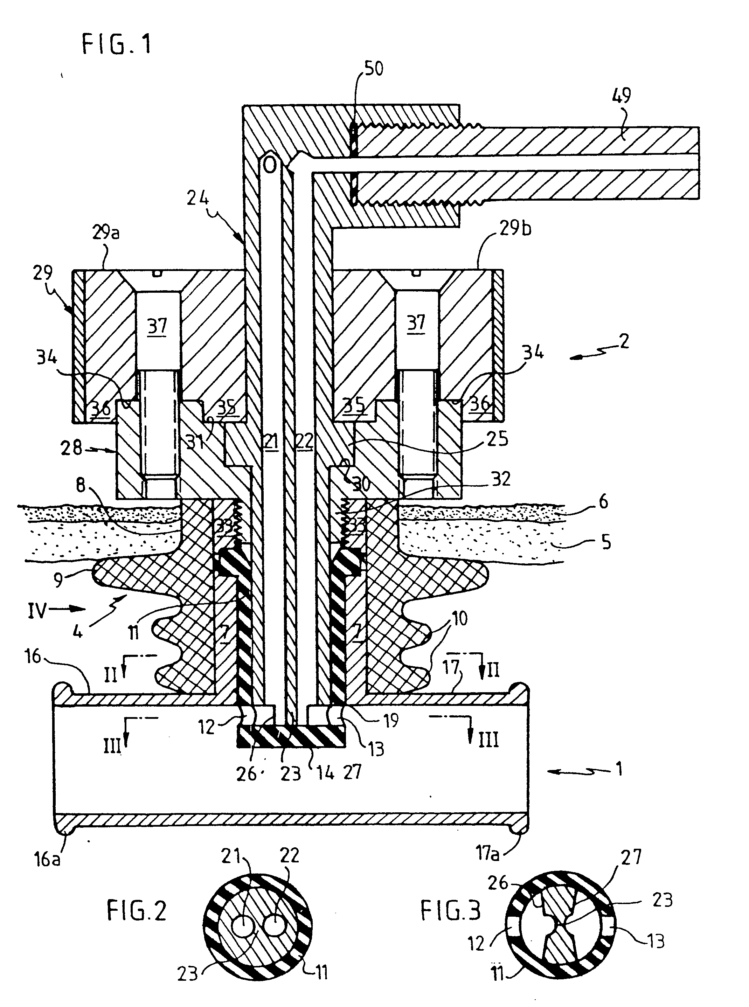

La différence entre l'élément d'obturation 11a et l'élément d'obturation 11 représenté aux fig. 1, 4 et 5 consiste essentiellement en ce que l'élément 11a présente:

– une base de fermeture 14a qui a une configuration arquée délimitée transversalement, à savoir perpendiculairement au sens de l'écoulement sanguin, par deux projections latérales 14b sensiblement triangulaires qui sont alignées avec la paroi latérale de l'élément d'obturation 11a: cette configuration axiale, qui s'adapte au profil intérieur du dispositif implantable 1, a pour but de réduire la turbulence due à l'immersion de la base de l'élément d'obturation dans le courant sanguin,
– des orifices latéraux 12a et 13a de communication avec le courant sanguin, qui sont oblongs et ménagés avec l'axe de l'écoulement sanguin de ces orifices 12a et 13a incliné, de préférence de 60°, de rapport à l'axe de l'élément d'obturation 11a et passant sensiblement par le point d'intersection de la base 14a avec la paroi latérale de cet élément tubulaire 11a: l'emploi de ces orifices oblongs et inclinés facilite l'écoulement sanguin et augmente le débit.

En outre, bien qu'on ait représenté l'élément d'obturation tubulaire comme étant constitué par une pièce cylindrique, il est également possible de donner à cet élément tubulaire une légère conicité.

Un mécanisme de commande de l'écoulement sanguin 2, réalisé en titane ou en acier inoxydable, par exemple, et comportant deux chambres tubulaires d'écoulement 21 et 22 délimitées par une cloison centrale longitudinale 23 et par une enveloppe 24 extérieure commune à ces chambres 21 et 22, coopère avec le segment central 7 du dispositif percutané 1 et l'élément d'obturation tubulaire 11.

A cet effet, ladite enveloppe 24 des chambres d'écoulement sanguin 21 et 22 présente, conformément à l'invention, une collerette annulaire 25 ménagée sensiblement à la partie médiane de ces chambres 21 et 22 qui se prolongent donc bilatéralement, par rapport à cette collerette 25, dans une partie distale et une partie proximale, laquelle collerette 25 est destinée à coopérer avec des moyens de blocage et d'entraînement desdites chambres d'écoulement 21 et 22 à l'intérieur dudit élément d'obturation tubulaire 11, qui est ainsi allongé élastiquement par la poussée axiale de ladite partie distale du mécanisme de commande 2 sur la base 14 de l'élément d'obturation 11: de cette manière la base 14 de ce dernier est immergée dans le courant sanguin avec les orifices 12 et 13 d'aspiration et de réinjection.

Lorsque ledit mécanisme de commande 2 de l'écoulement sanguin est retiré, l'élément d'obturation 11 reprend ses dimensions initiales sous l'action de la contrainte due à son allongement élastique.

La communication entre lesdits deux orifices 12 et 13 d'aspiration et de réinjection avec les chambres tubulaires d'écoulement 21 et 22, respectivement, est obtenue à l'aide de deux encoches transversales diamétralement opposées 26 et 27 qui sont ménagées au niveau de l'extrémité libre de la partie distale desdites chambres 21 et 22 sur une hauteur au moins égale au diamètre desdits orifices 12 et 13.

Avantageusement, les deux encoches 26 et 27 présentent une forme en V à grande ouverture, de préférence comprise entre 150 et 180°, ce qui a pour résultat d'assurer en permanence la communication avec les orifices d'aspiration et de réinjection 12 et 13, même si les chambres tubulaires d'écoulement 21 et 22 ne sont que très approximativement orientées, d'où une grande facilité d'emploi pour l'opérateur qui ne nécessite pas d'outils ou de moyens d'alignement.

En ce qui concerne lesdits moyens de blocage et d'entraînement des chambres d'écoulement 21 et 22, ils sont constitués par un écrou moleté 28 et par une bague de fixation 29.

Dans le corps cylindrique de l'écrou 28 est ménagé un premier siège central 30 destiné à loger ladite collerette 25 faisant saillie de l'enveloppe 24 des chambres d'écoulement 21 et 22, et d'un deuxième siège central 31 superposé au premier et de plus grand diamètre; du côté opposé à ces premier et deuxième sièges 30 et 31 et en communication avec ces derniers, l'écrou 28 comporte une partie en saillie centrale 32 qui est filetée sur sa surface externe et qui est destinée à être vissée dans une partie taraudée 33 se trouvant au-dessus de ladite rainure circulaire 18 du segment central 7 du dispositif percutané 1.

La bague de fixation 29, qui est coaxiale et superposée par rapport audit écrou moleté 28, se compose de deux demi-bagues 29a et 29b comportant chacune sur la face transversale qui regarde vers ce dernier, une rainure semi-circulaire 34 délimitant centralement une partie 35 destinée à être logée et centrée dans ledit deuxième siège de centrage 31 de cette bague composite 29 et à emprisonner, avec ledit écrou 28, ladite collerette 25 des chambres tubulaires d'écoulement 21 et 22 dans ledit premier siège 30.

Latéralement la rainure 34 délimite une partie 36 qui enveloppe partiellement l'écrou 28.

L'écrou 28 et la bague de fixation composite 29 sont solidarisés entre eux par des vis 37 notamment.

Il y a lieu de remarquer que le vissage dudit écrou 28 dans le segment central 7 du dispositif percutané 1 est rendu très facile et précis grâce à la partie distale du mécanisme de commande 2 de l'écoulement sanguin qui guide et aligne parfaitement l'écrou 28 dans son logement.

Un couvercle 3 (cf. la fig. 4) permet d'obturer le dispositif percutané 1 entre deux accès à la dialyse rénale.

Ce couvercle 3 comporte une plaquette d'obturation 38 destinée à couvrir le segment central 7 avec son revêtement composite 4.

Un joint annulaire d'étanchéité 39 peut être logé, à force, dans une rainure circulaire ménagée dans la face d'obturation de ladite plaquette 38, lequel joint 39 est avantageusement réalisé en élastomère.

A partir de la plaquette 38 fait saillie, vers l'intérieur dudit segment central 7, une première partie centrale cylindrique 40 filetée sur sa surface extérieure et destinée à être vissée dans ladite partie taraudée 33.

Une deuxième partie cylindrique centrale 41, de plus petit diamètre par rapport à la première, fait saillie à partir de cette dernière et se projette à l'intérieur dudit élément d'obturation tubulaire 11. Cette deuxième partie en saillie 41 permet l'alignement et le centrage d'un manchon de pression 42, qui entoure la partie en saillie 41 et qui est inséré à force dans l'élément d'obturation 11, en sorte que ce dernier est comprimé latéralement contre la paroi du segment central 7 et le manchon de pression 42 est bloqué en rotation: de cette façon il est possible de chasser vers le courant sanguin délimité par les segments latéraux 16 et 17 la mince pellicule de sang qui a pu éventuellement pénétrer entre les deux pièces 11 et 7.

Une troisième partie cylindrique centrale 43, faisant saillie à partir de ladite deuxième partie en saillie 41 et présentant un diamètre inférieur par rapport à cette dernière, est entourée par une rondelle 44 de diamètre supérieur à celui de la deuxième partie en saillie 41.

Cette rondelle 44 est maintenue en position contre la partie en saillie 41 par un évasement 45 de l'extrémité distale de ladite troisième partie en saillie 43, cet évasement 45 étant obtenu par sertissage ou par formage à chaud selon que le couvercle 3 est métallique ou réalisé en une matière plastique.

Une rainure cylindrique 46 est ménagée à la partie distale dudit manchon de pression 2, qui se prolonge jusqu'à toucher la base 14 de l'élément d'obturation 11, dans la paroi interne de ce manchon 42, et définit dans cette dernière un épaulement 47 contre lequel vient s'appliquer ladite rondelle 44: de cette façon, le manchon 42 est emprisonné entre la première partie en saillie 40 et la rondelle 44.

Les dimensions desdites parties en saillie 40, 41 et 43 et dudit manchon 42 sont telles que la force axiale de pression exercée sur la base 14 de l'élément d'obturation tubulaire 11 qui, à partir de la position de repos indiquée à la fig. 5, est portée par le couvercle 3 en position d'alignement avec la base 19 dudit segment central 7 (cf. la fig. 4), de manière à être tangente à la limite supérieure de la lumière 48 du shunt sanguin défini par les segments latéraux 16 et 17 du dispositif percutané 1.

Lorsqu'on désire effectuer l'opération de dialyse rénale, on introduit, après avoir ôté le couvercle 3, le mécanisme de commande de l'écoulement sanguin 2, à savoir la partie distale des chambres tubulaires d'écoulement 21 et 22, à l'intérieur de l'élément d'obturation tubulaire 11 en vissant l'écrou 28 dans le segment central 7 du dispositif percutané 1.

Pendant le vissage, l'extrémité de ladite partie distale des chambres d'écoulement 21 et 22 vient en appui sur la base 14 de l'élément d'obturation 11, ce qui cause l'allongement élastique de ce dernier et la mise en contact des orifices d'aspiration et de réinjection 12 et 13, et donc des chambres 21 et 22, par l'intermédiaire des encoches 26 et 27, avec le sang contenu dans les segments latéraux 16 et 17.

La liaison entre les chambres 21 et 22 et le dispositif d'épuration (non représenté) se fait à l'aide de deux embouts 49 qui sont placés l'un derrière l'autre et dont seulement un est visible dans le dessin, l'étanchéité entre ces embouts 49 et les chambres tubulaires d'écoulement 21 et 22 étant assurée par des joints 50.

Ces embouts 49 ne sont pas parallèles, mais convergent vers les chambres tubulaires d'écoulement 21 et 22.

En ce qui concerne le remplacement éventuel de l'élément d'obturation 11 du dispositif percutané 1, celui-ci est effectué d'une manière très simple: on fait sortir la collerette 15 et les ergots 20 de leurs logements par déformation élastique de l'élément d'obturation 11 après avoir pincé de l'extérieur le shunt artério-veineux se trouvant à droite et à gauche du segment central 7 du dispositif percutané 1, dont l'implantation est bien connue du malade et/ou de l'opérateur: généralement ce dispositif 1 est implanté au niveau du biceps perpendiculairement à ce muscle.

Ce pincement est nécessaire pour minimiser la perte de sang pendant l'enlèvement d'un élément d'obturation usé et son remplacement par un élément d'obturation nouveau.

Bien qu'on ait représenté le mécanisme de commande de l'écoulement sanguin comme étant pourvu, de préférence, de deux chambres (ou conduits) d'écoulement, il va de soi qu'on peut utiliser en variante aussi un mécanisme de commande pourvu d'une chambre (ou d'un conduit) unique.

**Revendications**

1. Dispositif d'accès atraumatique au circuit sanguin, du type comportant en combinaison:
   – un dispositif implantable (1) pourvu d'un segment (7) tubulaire d'accès à un vaisseau sanguin, ledit segment dépassant légèrement de la surface cutanée,
   – un élément d'obturation en matière élastique de ce segment (7),
   – un mécanisme de commande (2) de l'écoulement sanguin à travers cet élément d'obturation, ayant une partie proximale et une partie distale, ou, lorsque ce mécanisme est retiré,
   – un couvercle (3) de fermeture dudit segment tubulaire d'accès au sang,
   lequel dispositif est caractérisé en ce que ledit élément d'obturation élastique est constitué par une pièce tubulaire (11) réalisée en une matière élastique hémocompatible insérée à l'intérieur dudit segment (7) d'accès au sang, lequel élément d'obturation (11) est fermé à sa base (14) et est pourvu d'au moins un orifice latéral (12, 13) de communication avec ledit vaisseau sanguin et présente des moyens de retenue, les orifices latéraux (12, 13) de l'élément d'obturation étant obturés par la paroi de ce segment (7) en l'absence dudit mécanisme de commande et ledit élément d'obturation tubulaire (11) étant allongé élastiquement, lorsque le mécanisme de commande est présent, par la poussée axiale de ladite partie distale de ce dernier sur la base (14) de l'élément d'obturation, de manière à immerger dans le courant sanguin cette base et lesdits orifices (12 et 13), tandis que l'élément d'obturation (11) reprend ses dimensions initiales sous l'action de la contrainte due à son allongement élastique lorsque ledit mécanisme de commande (2) de l'écoulement sanguin est retiré.

2. Dispositif d'accès atraumatique au circuit sanguin selon la revendication 1, caractérisé en ce que lesdits moyens de retenue de l'élément d'obturation tubulaire (11) sont constitués de préférence par une colerette annulaire (15) ménagée autour de l'ouverture d'accès à cet élément d'obturation, laquelle collerette annulaire est pourvue d'une pluralité d'ergots transversaux (20) de blocage en rotation ménagés dans l'épaisseur de la collerette, et en ce que le segment tubulaire (7) du dispositif implantable (1) présente une rainure circulaire (18) dans laquelle est logée ladite collerette de retenue (15) de l'élément d'obturation (11), lesdits ergots transversaux (20) étant logés à leur tour dans des orifices percés dans la zone de la paroi dudit segment tubulaire (7) qui correspond à ladite rainure circulaire (18), laquelle rainure circulaire est ménagée dans la paroi interne du segment tubulaire (7) à une distance de l'orifice d'accès à ce segment tubulaire telle que, en l'absence d'application dudit mécanisme de commande (2) ou dudit couvercle (3), la base (14) dudit élément d'obturation (11) est légèrement rentrante par rapport à la base (19) dudit segment tubulaire (7).

3. Dispositif d'accès atraumatique au circuit sanguin selon la revendication 1, caractérisé en ce que lesdits orifices latéraux (12, 13) de communication avec ledit vaisseau sanguin sont ménagés sensiblement au niveau de l'intersection de la base (14) de fermeture de l'élément d'obturation (11) avec la paroi latérale de ce dernier.

4. Dispositif d'accès atraumatique au circuit sanguin selon la revendication 3, caractérisé en ce que lesdits orifices latéraux (12a, 13a) sont oblongs et ménagés avec l'axe d'écoulement sanguin de ces orifices (12a, 13a) incliné, de préférence de 60°, par rapport à l'axe de l'élément d'obturation tubulaire (11a) et passant sensiblement par le point d'intersection de ladite base (14a) avec ladite paroi latérale.

5. Dispositif d'accès atraumatique au circuit sanguin selon la revendication 1, caractérisé en ce que la base (14a) de fermeture de l'élément d'obturation tubulaire (11a) présente extérieurement une configuration arquée destinée à réduire la turbulence due à l'immersion de cette base dans le courant sanguin, laquelle configuration arquée est délimitée transversalement à savoir perpendiculairement au sens de l'écoulement sanguin, par deux projections latérales (14b) sensiblement triangulaires qui sont alignées avec la paroi latérale de l'élément d'obturation (11a).

6. Dispositif d'accès atraumatique au circuit sanguin selon la revendication 1, du type comportant un mécanisme de commande (2) de l'écoulement sanguin qui comprend deux chambres tubulaires d'écoulement (21, 22) communiquant avec deux embouts (49) reliés à un dispositif d'épuration sanguine qui sont pourvues de deux encoches (26, 27) diamétralement opposées, notamment en forme de V à grande ouverture, et qui sont solidarisées entre elles par une cloison longitudinale centrale (23) et une enveloppe extérieure (24) commune à ces chambres d'écoulement (21, 22), caractérisé en ce que l'enveloppe (24) desdites deux chambres d'écoulement comporte une collerette annulaire (25), ménagée sensiblement à sa partie médiane, par rapport à laquelle ces deux chambres d'écoulement se prolongent bilatéralement dans lesdites parties distale et proximale, laquelle collerette (25) est destinée à coopérer avec des moyens de blocage des chambres tubulaires (21 et 22) et d'entraînement par roto-translation de la partie distale de ces dernières à l'intérieur dudit élément d'obturation tubulaire élastique (11).

7. Dispositif d'accès atraumatique au circuit sanguin selon la revendication 6, caractérisé en ce que lesdits moyens de blocage et d'entraînement des chambres tubulaires d'écoulement (21 et 22) sont constitués par un écrou moleté (28), dont le corps cylindrique comporte un premier siège central (30) de logement de ladite collerette (25) faisant saillie de l'enveloppe (24) des chambres d'écoulement (21 et 22), un deuxième siège central de centrage (31) superposé au premier et de plus grand diamètre, et une partie en saillie centrale (32), disposée du côté opposé auxdits premier et deuxième sièges (30 et 31), laquelle est en communication avec ces derniers et filetée sur sa surface extérieure destinée à être vissée dans une partie taraudée (33) dudit segment tubulaire (7) du dispositif implantable (1) se trouvant au-dessus de ladite rainure circulaire (18), ainsi que par une bague de fixation (29), coaxiale et superposée par rapport audit écrou (28), qui se compose de deux demi-bagues (29a et 29b) comportant chacune, sur une face transversale, ne rainure semi-circulaire (34) délimitant centralement une partie (35) destinée à être logée et centrée dans ledit deuxième siège (31) de centrage de cette bague composite (29) et à emprisonner, avec ledit écrou (28), ladite collerette (25) des chambres d'écoulement (21 et 22) dans ledit premier siège (30), tandis que latéralement ladite rainure semi-circulaire (34) délimite une partie (36) destinée à envelopper partiellement ledit écrou (28), la solidarisation entre les deux demi-bagues de fixation (29a et 29b) et l'écrou (28) étant obtenue à l'aide de vis (37) notamment.

8. Dispositif d'accès atraumatique au circuit sanguin selon la revendication 1, caractérisé en ce que ledit couvercle (3) comporte une plaquette d'obturation (38) destinée à couvrir le segment tubulaire (7) du dispositif implantable (1), ainsi que son revêtement (4), et une première partie cylindrique centrale (40) qui fait saillie à partir de ladite plaquette (38) et qui est filetée et destinée à être vissée dans ladite partie taraudée (33) du segment tubulaire (7).

9. Dispositif d'accès atraumatique au circuit sanguin selon la revendication 8, caractérisé en ce que le couvercle (3) comporte une deuxième partie cylindrique centrale en saillie (41), de plus petit diamètre par rapport à ladite première partie en saillie filetée (40), qui se projette, à partir de cette dernière, à l'intérieur dudit élément d'obturation tubulaire (11) et qui est entourée par un manchon de pression (42) se prolongeant jusqu'à la base (14) de cet élément d'obturation tubulaire (11) et étant inséré à force dans ce dernier, lequel manchon (42) est donc immobilisé en rotation et comprime la paroi latérale dudit élément d'obturation (11) contre la paroi dudit segment tubulaire (7) du dispositif implantable (1), tandis qu'il permet la libre rotation de ladite deuxième partie en saillie (41) à l'intérieur de ce même manchon (42).

10. Dispositif d'accès atraumatique au circuit sanguin selon la revendication 9, caractérisé en ce que le couvercle (3) présente une troisième partie cylindrique centrale (43) qui fait saillie à partir de ladite deuxième partie en saillie (41) et qui présente un plus petit diamètre par rapport à cette dernière, laquelle troisième partie en saillie (43) est entourée par une rondelle de retenue (44), de diamètre supérieur à celui de la deuxième partie en saillie (41), qui est maintenue contre cette dernière par un évasement ou élargissement (45) de l'extrémité distale de ladite troisième partie en saillie (43), réalisée par sertissage ou par formage à chaud de cette extrémité notamment, laquelle rondelle (44) est logée dans une rainure cylindrique (46) ménagée à la partie distale dudit manchon de pression (42), dans la paroi interne de ce dernier, et définissant dans cette même paroi un épaulement (47) contre lequel est appliquée ladite rondelle (44) qui tient en position ledit manchon de pression (42).

11. Dispositif d'accès atraumatique au circuit sanguin selon la revendication 8, caractérisé en ce que la plaquette d'obturation (38) dudit couvercle (3) comporte un joint d'étanchéité annulaire (39) logé à force dans une rainure circulaire ménagée dans la face d'obturation de la plaquette (38).

12. Dispositif d'accès atraumatique au circuit sanguin selon la revendication 1, du type comportant en outre un revêtement en un matériau poreux (4) colonisable par croissance de tissus et entourant au moins partiellement ledit dispositif implantable (1), caractérisé en ce que ledit revêtement poreux (4) est constitué par un matériau composite carbone-carbone du type comportant une armature constituée par un substrat poreux en fibres de carbone noyée dans une matrice carbonée de densification de ce substrat fibreux et de liaison entre les fibres de carbone, dans lequel matériau composite carbone-carbone le substrat poreux en fibres de carbone est densifié seulement en partie par ladite matrice carbonée de façon à obtenir un matériau composite carbone-carbone poreux.

13. Dispositif d'accès atraumatique au circuit sanguin selon la revendication 12, caractérisé en ce que la porosité dudit matériau composite est de préférence comprise entre 40 et 50% de la porosité initiale dudit substrat de fibres de carbone.

14. Dispositif d'accès atraumatique au circuit sanguin selon la revendication 12, caractérisé en ce que ledit revêtement poreux (4) entoure complètement au moins ledit segment tubulaire (7) du dispositif implantable (1) sur toute sa longueur, y compris la partie de ce dernier qui dépasse légèrement de l'épiderme (6), et en ce qu'il comporte une partie proximale sensiblement cylindrique (8), dont la hauteur est légèrement supérieure à l'épaisseur totale du derme (5) et de l'épiderme (6) et qui est suivie d'une partie intermédiaire en forme de collerette circulaire (9) assurant l'ancrage biologique principal et disposée juste sous le derme (5), et d'une partie distale comportant une pluralité de nervures circulaires (10) assurant un ancrage biologique supplémentaire.

15. Dispositif d'accès atraumatique au circuit sanguin selon la revendication 14, caractérisé en ce que la partie proximale sensiblement cylindrique (8) dudit revêtement poreux (4) en composite carbone-carbone comporte une pellicule (4a) en carbone pyrolytique massif, étanche et non poreux, de protection contre la pénétration d'agents nuisibles tels que poussière, liquides ou autres, laquelle pellicule (4a) est appliquée sur la portion exposée du revêtement (4) parallèle à l'épiderme (6) ainsi que sur une petite portion de sa surface latérale extérieure perpendiculaire à l'épiderme (6).

**Patentansprüche**

1. Vorrichtung für atraumatischen Zugang zum Blutkreislauf, von der Art, die in Kombination umfasst:
– einer implantierbaren Vorrichtung (1), versehen mit einem röhrenförmigen Segment (7) für Zugang zu einem Blutgefäss, wobei das genannte Segment die Hautoberfläche leicht übersteigt,
– ein Dichtungselement aus elastischem Material dieses Segments (7),
– einen Befehlsmechanismus (2) des Abzuges von Blut durch dieses Dichtungselement, mit einem nahen Teil und einem ferner liegenden Teil, oder, wenn dieser Mechanismus zurückgezogen ist,
– einem Verschlussdeckel (3) genannten röhrenförmigen Segments für Zugang zum Blut,
wobei diese Vorrichtung dadurch gekennzeichnet ist, dass das genannte elastische Dichtungselement besteht aus einem röhrenförmigen Stück (11), hergestellt aus elastischem hämokompatiblem Material, eingeführt ins Innere genannten Segmentes (7) für Zugang zum Blut, wobei das Dichtungselement (11) an seiner Basis (14) geschlossen ist und versehen ist mit wenigstens einer seitlichen Öffnung (12, 13) der Kommunikation mit genanntem Blutgefäss, und Mittel zum Zurückhalten besitzt, wobei die seitlichen Öffnungen (12, 13) des Dichtungselements abgedichtet sind durch die Wand dieses Segmentes (7) in Abwesenheit des genannten Befehlsmechanismus und wobei das genannte röhrenförmige Dichtungselement (11) elastisch verlängert ist, wenn der Befehlsmechanismus vorhanden ist, durch den axialen Druck des genannten ferner liegenden Teils dieses letzteren auf die Basis (14) des Dichtungselements, in der Weise, dass diese Basis und die genannten Öffnungen (12, 13) in den Blutstrom eingetaucht werden, während das Dichtungselement (11) seine anfänglichen Dimensionen wieder einnimmt unter der Wirkung der Beanspruchung aufgrund seiner elastischen Verlängerung, wenn der genannte Befehlsmechanismus (2) des Blutabzuges zurückgezogen ist.

2. Vorrichtung für atraumatischen Zugang zum Blutkreislauf gemäss Anspruch 1, dadurch gekennzeichnet, dass die genannten Mittel zur Zurückhaltung des röhrenförmigen Dichtungselements (11) vorzugsweise bestehen aus einem ringförmigen Flansch (15), angeordnet um die Öffnung des Zugangs zu diesem Dichtungselement, wobei genannter ringförmiger Flansch versehen ist mit einer Vielzahl von transversalen Vorsprüngen (20) der Blockierung der Rotation, angeordnet in der Dicke des Flansches, und wobei das röhrenförmige Segment (7) der implantierbaren Vorrichtung (1) eine kreisförmige Rille (18) besitzt, in welcher genannter Rückhaltungsflansch (15) des Dichtungselements (11) angeordnet ist, wobei die genannten transversalen Vorsprünge (20) angeordnet sind bei ihrer Umdrehung in durchbohrten Öffnungen in der Zone der Wand genannten röhrenförmigen Segmentes (7), die der genannten kreisförmigen Rille (18) entspricht, wobei die genannte kreisförmige Rille angeordnet ist in der inneren Wand des röhrenförmigen Segmentes (7) in einem Abstand von der Zugangsöffnung zu diesem röhrenförmigen Segment dergestalt, dass in Abwesenheit der Anwendung genannten Befehlsmechanismus (2) oder genannten Deckels (3) die Basis (14) genannten Dichtungselements (11) leicht zurückweichend ist mit Bezug auf die Basis (19) genannten röhrenförmigen Segmentes (7).

3. Vorrichtung für atraumatischen Zugang zum Blutkreislauf gemäss Anspruch 1, dadurch gekennzeichnet, dass die genannten seitlichen Öffnungen (12, 13) der Kommunikation mit dem genannten Blutgefäss, im wesentlichen angeordnet sind auf der Ebene des Schnittpunktes der Basis (14) des Verschlusses des Dichtungselements (11) mit der seitlichen Wand des letzteren.

4. Vorrichtung für atraumatischen Zugang zum Blutkreislauf gemäss Anspruch 3, dadurch gekennzeichnet, dass die genannten seitlichen Öffnungen (12a, 13a) länglich sind und dergestalt angeordnet sind, mit der Achse des Blutstromes dieser Öffnungen (12a, 13a) geneigt, vorzugsweise um 60°, mit Bezug auf die Achse des röhrenförmigen Dichtungselements (11a) und im wesentlichen durch den Punkt des Schnittpunktes der genannten Basis (14a) mit genannter seitlicher Wand hindurchgehen.

5. Vorrichtung für atraumatischen Zugang zum Blutkreislauf gemäss Anspruch 1, dadurch gekennzeichnet, dass die Basis (14a) des Verschlusses des röhrenförmigen Dichtungselements (11a) äusserlich eine bogenförmige Konfiguration besitzt, die dazu bestimmt ist, die Turbulenz zu reduzieren, die verursacht wird durch das Eintauchen dieser Basis in den Blutkreislauf, wobei diese bogenförmige Konfiguration transversal begrenzt ist, nämlich senkrecht zur Richtung des Blutstromes, durch zwei seitliche Projektionen (14b) von im wesentlichen dreieckiger Form, die ausgerichtet sind, mit der seitlichen Wand des Dichtungselements (11a).

6. Vorrichtung für atraumatischen Zugang zum Blutkreislauf gemäss Anspruch 1, von der Art, die einen Befehlsmechanismus (2) des Blutabzuges umfasst, der besteht aus zwei röhrenförmigen Abzugskammern (21, 22), die mit zwei Abatzstücken (49) kommunizieren, die verbunden sind mit einer Vorrichtung zur Blutreinigung, die versehen sind mit zwei einander diametral gegenübergesetzten Nuten (26, 27), insbesondere in Form eines grossen V bei grosser Öffnung, und die untereinander verbunden sind durch eine zentrale longitudinale Wand (23) und eine äussere Umhüllung (24), die diesen Abzugskammern (21, 22) gemeinsam ist, dadurch gekennzeichnet, dass die Umhüllung (24) genannter zweier Abzugskammern einen ringförmigen Flansch (25) umfasst, angeordnet im wesentlichen in seinem Mittelteil mit Bezug auf welchen diese zwei Abzugskammern sich bilateral verlängern in genannten ferner liegenden und näher liegenden Teilen, wobei genannter Flansch (25) dazu bestimmt ist, mit den Blockierungsmitteln der röhrenförmigen Kammern (21, 22) zu kooperieren und dem Antrieb durch rotierende Übersetzung des ferner liegenden Teils dieses letzteren zum Inneren genannten elastischen röhrenförmigen Dichtungselements (11).

7. Vorrichtung für atraumatischen Zugang zum Blutkreislauf gemäss Anspruch 6, dadurch gekennzeichnet, dass die genannten Blockierungsmittel und Antriebsmittel der röhrenförmigen Abzugskammern (21, 22) dargestellt sind durch eine gerändelte Schraubenmutter (28), deren zylindrischer Körper einen ersten zentralen Sitz (30) der Anordnung genannten Flansches (25) besitzt, der die Umhüllung (24) der Abzugskammern (21, 22) vorspringend macht, einen zweiten zentralen Zentrierungssitz (31) dem ersten übereinander angeordnet und von grösserem Durchmesser, und einen zentralen vorspringenden Teil (32), angeordnet an der Seite, die genannten ersten zweiten Sitzen (30, 31) gegenüberliegt, die in Verbindung ist mit den letzteren, geschnitten ist auf ihrer äusseren Oberfläche, dazu bestimmt, angeschraubt zu werden in einem schraubenförmig ausgebohrten Teil (33) des genannten röhrenförmigen Segments (7) der implantierbaren Vorrichtung (1), die sich oberhalb genannter kreisförmiger Rille (18) befindet, ebenso wie durch einen Befestigungsring (29), koaxial und übereinander angeordnet mit Bezug auf genannte Schraubenmutter (28), der sich aus zwei Halbringen (29a und 29b) zusammensetzt, von denen jeder, auf einer transversalen Fläche, eine halbkreisförmige Rille (34) besitzt, die zentral einen Teil (35) begrenzt, der dazu bestimmt ist, angeordnet und zentriert zu werden in genanntem zweitem Sitz (31) zur Zentrierung dieses zusammengesetzten Ringes (29) und zum Festhalten mit genannter Schraubenmutter (28) des genannten Flansches (25) der Abzugskammern (21, 22) in genanntem erstem Sitz (30), während seitlich die genannte halbkreisförmige Rille (34) einen Teil (36) begrenzt, der dazu bestimmt ist, genannte Schraubenmutter (28) teilweise zu umhüllen, wobei die Verbindung zwischen den beiden Halbringen zur Fixierung (29a und 29b) und der Schraubenmutter (28) erhalten wird mit Hilfe von besonderer Schraube (37).

8. Vorrichtung für atraumatischen Zugang zum Blutkreislauf gemäss Anspruch 1, dadurch gekennzeichnet, dass der genannte Deckel (3) eine Dichtungsplakette (38) umfasst, die dazu bestimmt ist, das röhrenförmige Segment (7) der implantierbaren Vorrichtung (1) zu bedecken, ebenso wie seine Verkleidungsschicht (4), und einen ersten zentralen zylindrischen Teil (40), der vorspringend ist, ausgehend von genannter kleiner Plakette (38), und der schraubengewindeförmig geschnitten ist, und dazu bestimmt ist, in genannten schraubenförmig ausgebohrten Teil (33) des röhrenförmigen Segments (7) eingeschraubt zu werden.

9. Vorrichtung für atraumatischen Zugang zum Blutkreislauf gemäss Anspruch 8, dadurch gekennzeichnet, dass der Deckel (3) einen zweiten zylindrischen zentralen vorspringenden Teil (41) umfasst, von kleinerem Durchmesser mit Bezug auf den genannten ersten vorspringenden schraubenförmig geschnittenen Teil (40), der sich vorstreckt, ausgehend von diesem letzteren, ins Innere genannten röhrenförmigen Dichtungselementes (11), und der umgeben ist von einer Druckmanschette (42), die sich bis zur Basis (14) dieses röhrenförmigen Dichtungselements (11) erstreckt, und mit Kraft in dieses letztere eingesteckt ist, wobei diese Manschette (42) also befestigt ist in Rotation und die seitliche Wand des genannten Dichtungselements (11) gegen die Wand genannten röhrenförmigen Segmentes (7) der implantierbaren Vorrichtung (1) umfasst, während es die freie Rotation genannten zweiten vorspringenden Teils (41) ins Innere dieser gleichen Manschette (42) erlaubt.

10. Vorrichtung für atraumatischen Zugang zum Blutkreislauf gemäss Anspruch 9, dadurch gekennzeichnet, dass der Deckel (3) einen dritten zentralen zylindrischen Teil umfasst, der vorspringt, ausgehend von genanntem zweitem vorspringendem Teil (41), und der einen kleineren Durchmesser besitzt mit Bezug auf diesen letzteren, wobei dieser dritte vorspringende Teil (43) umgeben ist von einem Rückhaltedichtungsring (44), von einem Durchmesser, der grösser ist als derjenige des zweiten vorspringenden Teils (41), der gegen diesen letzteren gehalten ist durch eine Ausweitung oder Vergrösserung (45) des äusseren Endes genannten dritten vorspringenden Teils (43), dargestellt durch Einfassung oder durch insbesondere Bildung in der Wärme dieses äusseren Endes, wobei der Dichtungsring (44) angeordnet ist in einer zylindrischen Rille (46), angeordnet im entfernteren Teil genannter Druckmanschette (42), in der inneren Wand dieser letzteren und in dieser selben Wand eine Schulter (47) bildet, gegen welche der genannte Dichtungsring (44) angedrückt wird, der die genannte Druckmanschette (42) in Position hält.

11. Vorrichtung für atraumatischen Zugang zum Blutkreislauf gemäss Anspruch 8, dadurch gekennzeichnet, dass die Verschlussplakette (38) genannten Deckels (3) ein ringförmiges Abdichtungsgelenk (39) umfasst, angeordnet mit Kraft in einer kreisförmigen Rille, angebracht auf der Oberfläche der Dichtungsplakette (38).

12. Vorrichtung für atraumatischen Zugang zum Blutkreislauf gemäss Anspruch 1, vom Typ umfassend unter anderem eine Umhüllung aus porösem Material (4), kollonisierbar durch Wachsen von Geweben, und wenigstens teilweise genannte implantierbare Vorrichtung (1) umhüllend, dadurch gekennzeichnet, dass genannte poröse Umhüllung (4) besteht aus einem Kohlenstoff-Kohlenstoff-Komposit-Material, von der Art, umfassend eine Verstärkung, dargestellt durch ein poröses Substrat aus Kohlenstoff-Fasern, eingebettet in eine Kohlenstoff-Verdichtungs-Matrix dieses Fasersubstrates und der Verbindung zwischen den Kohlenstoff-Fasern, wobei in dem Kohlenstoff-Kohlenstoff-Komposit-Material das poröse Substrat aus Kohlenstoff-Fasern nur teilweise verdichtet ist durch genannte kohlenstoffhaltige Matrix in der Weise um ein poröses Kohlenstoff-Kohlenstoff-Material zu erhalten.

13. Vorrichtung für atraumatischen Zugang zum Blutkreislauf gemäss Anspruch 12, dadurch gekennzeichnet, dass die Porosität genannten Komposit-Materials vorzugsweise zwischen 40 und 50% der anfänglichen Porosität genannten Substrates von Kohlenstoff-Fasern umfasst.

14. Vorrichtung für atraumatischen Zugang zum Blutkreislauf gemäss Anspruch 12, dadurch gekennzeichnet, dass die genannte poröse Umhüllung (4) vollständig wenigstens genanntes röhrenförmiges Segment (7) der implantierbaren Vorrichtung (1) auf seiner gesamten Länge umhüllt, die den Teil dieser letzteren umfasst, der die Haut (6) leicht übersteigt, und dass sie umfasst einen im wesentlichen zylindrischen proximalen Teil (8), deren Höhe leicht grösser ist als die Gesamtdicke der Haut (5) und der Oberhaut (6), und der gefolgt wird von einem Zwischenteil in Form eines kreisförmigen Flansches (9), der die hauptsächliche biologische Verankerung sicherstellt, und angeordnet ist direkt unter der Haut (5), und einen weiter entfernten Teil, der eine Vielzahl von ringförmigen Rippen (10) umfasst, die eine ergänzende biologische Verankerung sicherstellen.

15. Vorrichtung für atraumatischen Zugang zum Blutkreislauf gemäss Anspruch 14, dadurch gekennzeichnet, dass der im wesentlichen zylindrische naheliegende (proximale) Teil (8) genannter poröser Umhüllung (4) aus Kohlenstoff-Kohlenstoff-Komposit eine Schicht (4a) aus festem pyrolytischem Kohlenstoff, dicht und nicht porös, umfasst, zum Schutz gegen das Eindringen störender Agenzien wie Staub, Flüssigkeiten oder andere, wobei genannte Schicht (4a) angebracht ist auf dem exponierten Teil der Umhüllung (4) parallel zur Aussenhaut (6) ebenso wie auf einem kleinen Teil der seitlichen äusseren Oberfläche senkrecht zur Oberhaut (6).

**Claims**

1. Device for atraumatic access to the blood circuit comprising in combination:

– an implantable device (1) provided with a tubular segment (7) for access to a blood vessel, said segment extending slightly from the cutaneous surface,

– a resilient material element for closing this segment (7),

– a mechanism for controlling the blood flow through this closure element, having a proximal part and a distal part, or, when this mechanism is removed,

– a lid (3) for closing said tubular segment for access to the blood

which device is characterized in that the resilient closure element is formed by a tubular piece (11) made from a hemocompatible resilient material inserted inside said segment for access to the blood (7) which closure element (11) is closed at its base (14) and is provided with at least one lateral orifice (12, 13) for communication with said blood vessel and has retention means, the lateral orifices (12, 13) of the closure element being closed by the wall of this segment (7) in the absence of said control mechanism and said tubular closure element (11) being resiliently elongated, when the control mechanism is present, by the axial thrust of said distal part of this latter on the base (14) of the closure element so as to immerse this base and said orifices (12 and 13) in the blood stream, whereas the closure element (11) resumes its initial dimensions under the action of the stress due to its resilient elongation when said mechanism (2) for controlling the blood flow is withdrawn.

2. Device for atraumatic access to the blood circuit according to claim 1, characterized in that said means for retaining the tubular closure element (11) are preferably formed by an annular collar (15) formed about the access opening to this closure element, which annular collar is provided with a plurality of transverse studs (20) for locking against rotation formed in the thickness of the collar, and in that the tubular segment (7) of the implantable device (1) has a circular groove (18) in which is housed said collar (15) for retaining the closure element (11), said transverse studs (20) being in their turn housed in orifices formed in the zone of the wall of said tubular segment (7) which corresponds to said circular groove (18), which circular groove is formed in the internal wall of the tubular segment (7) at a distance from the access orifice to this tubular segment such that, in the absence of application of said control mechanism (2) or of said lid (3), the base (14) of said closure element (11) is slightly reentrant with respect to the base (19) of said tubular segment (7).

3. Device for atraumatic access to the blood circuit according to claim 1, characterized in that said lateral orifices (12, 13) for communication with said blood vessel are formed substantially at the level of the intersection of the closed base (14) of the closure element (11) with the lateral wall of this latter.

4. Device for atraumatic access to the blood circuit according to claim 3, characterized in that said lateral orifices (12a, 13a) are oblong and formed with the blood flow access of these orifices (12a, 13a) inclined, preferably by 60°, with respect to the axis of the tubular closure element (11a) and passing substantially through the point of intersection of said base (14a) with said lateral wall.

5. Device for atraumatic access to the blood circuit according to claim 1, characterized in that the closed base (14a) of the tubular closure element (11a) has externally an arcuate configuration for reducing the turbulence due to immersion of this base in the blood stream, which arcuate configuration is defined transversally, namely perpendicularly to the direction of the blood flow, by two lateral substantially triangular projections (14b) which are aligned with the lateral wall of the closure element (11a).

6. Device for atraumatic access to the blood circuit according to claim 1, of the type comprising a mechanism (2) for controlling the blood flow which includes two tubular flow chambers (21, 22) communicating with two end pieces (49) connected to a blood purifying device which are provided with two diametrically opposite notches (26, 27), particularly in the form of a V with wide opening, and which are joined together by a central lingitudinal dividing wall (23) and an outer casing (24) common to these flow chambers (21, 22), characterized in that the casing (24) of said two flow chambers has an annular collar (25) formed substantially in its median part, with respect to which these two flow chambers extend bilaterally into said distal and proximal parts, which collar (25) is intended to cooperate with means for blocking the tubular chambers (21 and 22) and for driving by roto-translation the distal part of these latter inside said resilient tubular closure element (11).

7. Device for atraumatic access to the blood circuit according to claim 6, characterized in that said means for blocking and driving the tubular flow chambers (21 and 22) are formed by a knurled nut (28), whose cylindrical body comprises a first central seat (30) for housing said collar (25) projecting from the casing (24) of the flow chambers (21 and 22), a second central centering seat (31) superimposed on the first one and of a larger diameter, and a central projecting part (32), disposed on the side opposite said first and second seats (30 and 31), which is in communcation with these latter and threaded over its external surface intended to be screwed into a tapped part (33) of said tubular segment (7) of the implantable device (1) situated above said circular groove (18), as well as by a coaxial fixing ring (29) superimposed with respect to said nut (28), which is formed of two semirings (29a and 29b) each comprising, on a transverse face, a semicircular groove (34) defining centrally a part (35) intended to be housed and centered in said second seat (31) for centering this composite ring (29) and to imprison, with said nut (28), said collar (25) of the flow chambers (21 and 22) in said first seat (30), whereas laterally said semicircular groove (34) defined a part (36) intended to partially envelope said nut (28) the two semifixing rings (29a and 29b) and the nut (29) being secured together by means of screws (37) more particularly.

8. Device for atraumatic access to the blood circuit according to claim 1, characterized in that said lid (3) includes a closure plate (38) intended to cover the tubular segment (7) of the implantable device (1), as well as its covering (4), and a first central cylindrical part (40) which projects from said blade (38) and which is threaded and intended to be screwed into said tapped part (33) of the tubular segment (7).

9. Device for atraumatic access to the blood circuit according to claim 8, characterized in that the lid (3) comprises a second central cylindrical projecting part (41), of a smaller diameter than said first threaded projecting part (40) which extends, from this latter, inside said tubular closure element (11) and which is surrounded by a pressure sleeve (42) extending as far as the base (4) of this tubular closure element (11) and being force fitted thereinside, which sleeve (42) is therefore immobilized against rotation and compresses the lateral wall of said closure element (11) against the wall of said tubular segment (7) of the implantable device (1), whereas it allows free rotation of said second projecting part inside this same sleeve (42).

10. Device for atraumatic access to the blood circuit according to claim 9, characterized in that the lid (3) has a third central cylindrical part (43) which projects from said second projecting part (41) and which has a smaller diameter than this latter, which third projecting part (43) is surrounded by a retention washer (44), of a diameter greater than that of the second projecting part (41), which is held against this latter by a widened portion (45) of the distal end of said third projecting part (43), obtained by crimping or by hot forming this end more particularly, which washer (44) is housed in a cylindrical groove (46) formed in the distal part of said pressure sleeve (42), in the internal wall thereof, and defining in this same wall a shoulder (47) against which said washer (44) is applied which holds said pressure sleeve (42) in position.

11. Device for atraumatic access to the blood circuit according to claim 8, characterized in that the closure plate (38) of said lid (3) comprises an annular seal (39) force fitted into a circular groove formed in the closure face of the plate (38).

12. Device for atraumatic access to the blood circuit according to claim 1, of the type further comprising a covering of a porous material (4) which may be colonized by tissue growth and surrounding said implantable device (1) at least partially, characterized in that said porous coating (4) is formed by a composite carbon-carbon material of the type having a framework formed by a porous substrate of carbon fibers embedded in a carbonaceous matrix for densifying this fibrous substrate and for connecting between the carbon fibers, in which composite carbon-carbon material the porous carbon fiber substrate is densified only partially by said carbonaceous matrix so as to obtain a porous composite carbon-carbon material.

13. Device for atraumatic access to the blood circuit according to claim 12, characterized in that the porosity of said composite material is preferably between 40% and 50% of the initial porosity of said carbon fiber substrate.

14. Device for atraumatic access to the blood circuit according to claim 12, characterized in that said porous coating (4) completely surrounds at least said tubular segment (7) of the implantable device (1) over the whole of its length, including the portion thereof which extends slightly from the epiderm (6) and in that it includes a substantially cylindrical proximal part (8) whose height is slightly greater than the total thickness of the derm (5) and of the epiderm (6) and which is followed by an intermediate part in the form of a circular collar (9) providing the main biological anchorage and disposed just under the derm (5), and a distal part comprising a plurality of circular ribs (10) providing additional biological anchorage.

15. Device for atraumatic access to the blood circuit according to claim 14, characterized in that the substantially cylindrical proximal part (8) of said composite carbon-carbon porous coating (4) comprises a sealing and non porous film (4a) of solid pyrolitic carbon for protection against the penetration of harmful agents such as dust, liquids or the like, which film (4a) is applied on the exposed portion of coating (4) parallel to the epiderm (5) as well as on a small portion of the external lateral surface perpendicular to the epiderm (5).

**FIG.1**

**FIG.2**

**FIG.3**

# FIG. 4

# FIG. 5

## FIG. 7

## FIG. 6

## FIG. 8